# NEW EUROPEAN PATENT SPECIFICATION

(11) **EP 0 365 660 B2**
(45) Date of publication and mention of the opposition decision: **19.01.2000**
(45) Mention of the grant of the patent: 08.03.1995
(21) Application number: 89905576.8
(22) Date of filing: 07.12.1988
(51) Int. Cl.: A61B 6/08

(54) **X-RAY TOMOGRAPHY APPARATUS HAVING A POSITION DETECTOR**
RöNTGENSTRAHLEN-TOMOGRAPHIEGERÄT MIT EINEM ORTUNGSDETEKTOR
APPAREIL DE TOMOGRAPHIE PAR RAYON X AYANT UN DETECTEUR DE POSITION

(30) Priority: 22.04.1988 US 185445
(43) Date of publication of application: 02.05.1990
(73) Proprietor: ANALOGIC CORPORATION, Peabody Massachusetts 01961 (US)
(72) Inventor: GORDON, Bernard, M., Magnolia, MA 01930 (US); ABENAIM, Daniel, Lynnfield, MA 01940 (US)
(74) Representative: Chettle, Adrian John
(86) International application number: US8804351
(87) International publication number: WO8910090

(56) References cited:
- EP-A- 0 008 264
- EP-A- 0 048 075
- EP-A- 0 050 510
- DE-A- 3 546 233
- FR-A- 2 331 934
- FR-A- 2 366 561

## Description

### BACKGROUND OF THE INVENTION

### Field of the Invention

The present invention generally relates to x-ray tomography apparatuses which operate while in motion relative to the object being x-rayed and, in particular, to the positional alignment of the moving portions of those apparatuses.

### Statement of the Prior Art

X-ray tomography apparatuses have been used for many years. The most commonly known form is a CAT scan (computerized axial tomography) which operates by taking multiple, cross-sectional, slice x-rays passing through the body. One form of CAT scan apparatus typically includes an x-ray source and an array of detectors mounted on opposite sides of an annular collar, which collar is made to rotate within an imaging plane and around the patient. During rotation many slice x-rays are taken from different angles within the imaging plane. The results are mathematically compiled by a process known as backprojection, to create a cross-sectional image of the body in that plane.

An important consideration in scanning is the accurate and consistent alignment of the tomography components and the patient both throughout the rotation and over the course of many scans and patients. Misalignment can negatively influence the data of an entire scan. In order to deal with this factor, current CAT scan apparatuses are typically very large and massive and include a heavy collar for mounting the tomography components. The handling of this weight requires additional mass and size in the remainder of the apparatus.

Apparatuses thus constructed are expensive and difficult to relocate. They require large amounts of floor space and thus cannot be used in space limited environments. A further disadvantage caused by this size and weight is the wear experienced in the moving parts thereof. Over time, this wear contributes to misalignment.

In another form of tomography apparatus known in the prior art, the x-ray source and an array of detectors are mounted on a "C" shaped frame which is typically cantilevered and manipulated over the patient. Although this apparatus can be manipulated over a wide range, the manipulation apparatus also requires large amounts of space and mass and suffers from misalignment problems.

EP-A-0008264 discloses a radiography apparatus having a rotatable member carrying an X-ray source and means for rotating the rotatable member around an axis of rotation during an X-ray scan.

The apparatus acquires positional information regarding the relative position of the X-ray source during a test rotation, and subsequently performs a scan using this previously acquired information.

EP-A-0050510 discloses a radiography apparatus for radiating an X-ray beam in a fan shape in a plane, wherein the distance between the X-ray tube and the living body that is to be treated is determined every given angle component of said fan in order to finally deliver an error compensated radiogram.

EP-A-0048075 discloses a radiography apparatus in which the distance between a rotatable radiation detector and the subject that is to be examined may be determined at every rotational position and may be adjusted in response thereto.

DE-A- 35 46 233 and US-A- 4703424 disclose X-ray tomography apparatus having position detecting means measuring the positional relationship between a rotatable support means and a ring shaped stationary reference means. Also disclosed is a method of numerically correcting for a shift of the centre of a computer tomography system.

According to the present invention there is provided a tomography X-ray apparatus having (a) rotatable support means for supporting at least an X-ray source for rotation during a tomography scan of a patient, and (b) X-ray detector means for sensing X-rays during the tomographic scan, said apparatus further comprising:
stationary reference means positioned relative to said rotatable support means so as to remain stationary as said rotatable support means rotates about said axis of rotation during a scan;
position detecting means measuring the positional relationship between the rotatable support means and the stationary reference means; and
generating means for generating data representative of the positional relationship between the rotatable support means and the stationary reference means so that said data can be used when backprojecting a scanned image of the object; characterised in that the stationary reference means is a ring, and in real time during a scan of the patient both the position detecting means measures the positional relationship between the rotationable support means and stationary reference means, and the generating means generates data representative of the said positional relationship.

Such an apparatus provides real time acquisition of positional data.

Accordingly, the present invention provides an improved X-ray tomography apparatus which monitors alignment of the tomography components for providing accurate scan data while requiring substantially less size and mass than comparable apparatuses. In a refinement of the present invention, the rotatable means defines a plane of rotation and the positional relationship is determined for the two orthogonal dimensions within the plane of rotation.

The present invention is illustratively described with respect to the accompanying drawings in which:
Fig. 1 is a front view of a CAT scan apparatus constructed in accordance with one embodiment of the present invention;
Fig. 1A is a partial detail drawing of one variation of the embodiment of Fig. 1;
Fig. 1B is an alternate partial detail drawing of one variation of the embodiment of Fig. 1;
Fig. 2 is a front view of a CAT scan apparatus constructed in accordance with another embodiment of the present invention;
Fig. 3 is a perspective view of a CAT scan apparatus constructed in accordance with yet another embodiment of the present invention; and
Fig. 4 is a detail view of a portion of the apparatus of Fig. 3.

### DETAILED DESCRIPTION OF THE DRAWINGS

The present application is generally related to the U.S. Patent Application of Bernard M. Gordon entitled X-RAY TOMOGRAPHY APPARATUS, Serial No. 160,657, filed February 26, 1988, and published as WO-A-8908269 on September 8, 1989, which, as the present application, is assigned to Analogic Corporation of Peabody, Massachusetts, and the disclosure of which is hereby incorporated by reference herein.

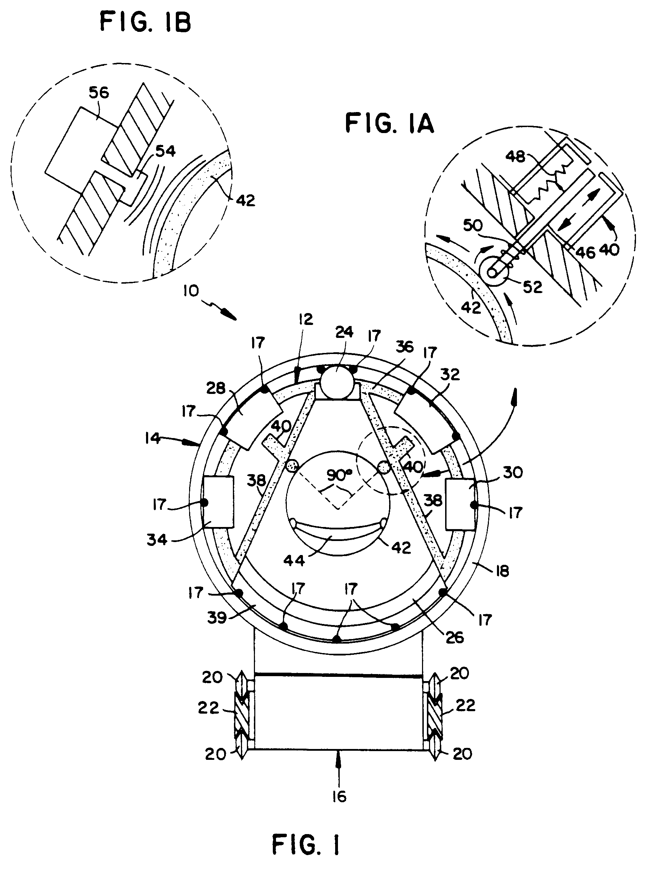

Fig. 1 shows a CAT scan apparatus 10 generally including a rotatable collar 12 bearing x-ray tomography components and a stationary support means 14. The support 14 includes a base section 16 for supporting the apparatus and a circular frame 18 for rotatably mounting the collar 12. The rotatable mounting may be accomplished by any suitable means including rollers or wheels 17 running on a track formed on the frame 18. The base 16 nominally includes means for relocating the apparatus, in the form of rollers 20 and a pair of support rails 22.

The collar 12 includes a multiplicity of components for performing the CAT scan operations. The essential components include a source of x-rays 24 and an array of x-ray detectors 26. The collar may further include various components depending upon the set-up of the apparatus. The present embodiment is configured to include a power supply 28 for the x-ray source 24, an electronics package 30 for providing data handling for the output of the detector array 26 and for providing control of the tomography collar 12, a power supply 32 for the electronics package 30, and a locomotion means 34, including a power supply, for causing the collar 12 to rotate within the circular frame 18. These various components are interconneted electrically as is necessary by wires which are not shown but may be run by any suitable routes, such as through hollow collar components 36.

Further included in the collar 12 are a pair of stiffening members 38 which are connected between the x-ray source 24 and the detector array 26. The members 38 are triangularly placed to take advantage of the stiffness of the triangular geometric structure. The detector array 26 is mounted on a third rigid member 39 which forms the third leg of the triangle.

Mounted on the stiffening members 38 are a pair of position detectors 40. Detectors 40 are located to detect the position of a reference ring 42 which is non-rotating. They are located orthogonally with respect to each other in order to detect the relative position between the rotating collar 12 and the reference ring 42 within the two dimensions of the imaginary plane of rotation of the collar 12. The reference ring 42 is shown affixed to a stationary reference such as the patient table 44. It may alternatively be affixed to the circular frame 18 or to a portion of the base 16. The reference ring 42 and patient table 44 are shown somewhat smaller in proportion than the collar 12 for purposes of clarity. Reference ring 42 would typically be almost as large as the circle of rotation defined by the stiffening members 38.

The detectors 40 may detect the ring 42 by any suitable means such as direct mechanical contact or reflected energy. In each case, the detector measures the distance between that detector and the reference ring 42. In addition one or more detectors may be counted to sense the relative position between the ring 42 and collar 12 in the dimension substantially perpendicular to the plane of rotation of the collar and the plane of the ring 42.

Fig. 1A shows the detail of one form of the detector 40 and its engagement with the reference ring 42. Included in the detectors 40 are a cam follower 46, for following the reference ring 42, and a variable resistor 48 controlled by the cam follower 46. The cam follower 46 is biased by a spring 50 and includes a cam roller 52 rotatably mounted at one end for engaging the reference ring 42. The variable resistor 48 provides an electrical signal in response to the mechanically detected position of the ring. A variable capacitor may also be used in place of the resistor 48.

Fig. 1B shows an alternate embodiment of the detector 40 which uses reflected energy to measure the distance to the ring 42. A sonic transducer 54 is shown connected to a controller 56. The controller causes the transducer 54 to transmit short pulses of sound at the ring 42 and listens for the energy reflected back from the ring. The time delay between transmission and reception indicates the distance from the ring 42. Although the use of sound is shown, other means of transmitted energy such as light and radio waves may be used. Readable coding may also be provided on the ring 42 to allow a measurement of angular position to be provided during scanning.

In operation, the apparatus 10 of Fig. 1 functions to constantly detect the relative position between the rotating collar 12 and the reference ring 42. As the collar 12 rotates, the distance between the ring 42 and the detectors 40 is constantly measured. This measurement is converted by the electronics package 30 into a digital reading which is used in the backprojection of x-ray data to produce an image of the scanned slice. The orthogonal placement of the two detectors with respect to each other provides complete data on the two dimensional or X-Y location of the rotating collar 12 within its imaginary plane of rotation which would be parallel to the surface of the drawing of Fig. 1.

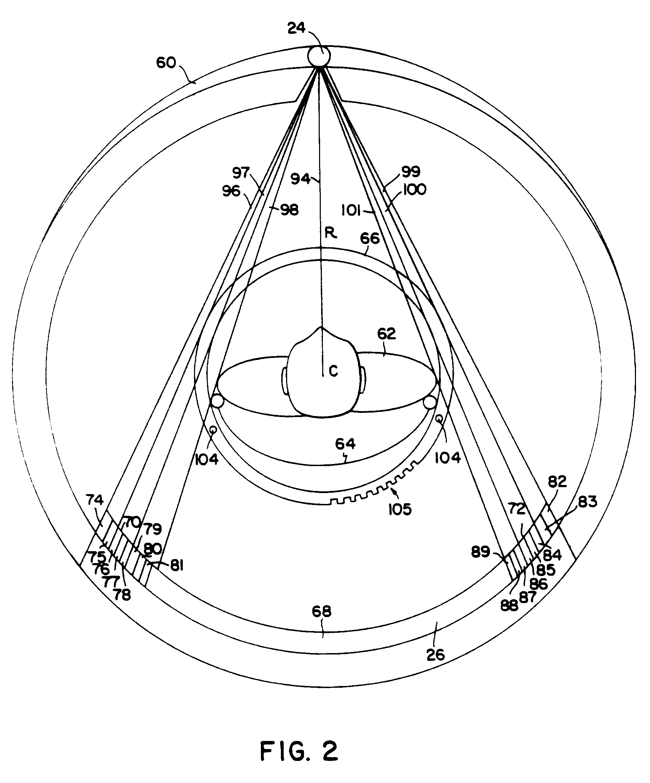

Fig. 2 shows another embodiment of the present invention wherein the relative position of the rotating collar is measured by means of x-ray detectors 26. Fig. 2 shows only the essential features of the CAT scan apparatus, including the collar 60 carrying the x-ray source 24 and the array of detectors. Also shown are a patient 62, a patient table 64, and a reference ring 66. Detector array 26 is shown to have three sections of detectors 68, 70, and 72. The detectors of section 68 serve the purpose of collecting data from the patient 62 and may be constructed in any suitable manner for this purpose. The additional detectors shown in sections 70 and 72 are used for locating the relative position of the reference ring 66 with respect to the x-ray source 24 and those sections 70,72 of detectors. Section 70 is shown to include individual detectors 74 through 81, and section 72 is shown to include individual detectors 82 through 89. The number of detectors shown in each section is not critical and may be varied in accordance with the design of the apparatus.

The reference ring 66 is constructed of x-ray translucent material and is located in the imaginary plane through which the x-rays travel from the source 24 to the detector array 26. Thusly, the x-rays used for collecting data from the patient 62 also pass through the ring 66 and are slightly attenuated thereby. Because of the geometry of the reference ring 66, x-rays passing through the center of the subject 62, as exemplified by center radial line 94, are attenuated the least amount by the ring 66. This attenuation increases slightly as the angle of the x-ray radial line moves from the center 94 out towards the edges. This condition is acceptable because the information content within the patient also decreases depending upon the angle of the radial line from the center. The most complex information of the patient is located nearest the center and decreases as the angle moves past the edge of the head and still further as the angle moves past the boundaries of the torso. Thusly, increased attentuation near the outer edges of the patient is acceptable and even desirable as this decreases the dynamic range of the x-ray signals which must be measured by the detectors. Any suitable material may be used for the ring 66 such as fiberglass and other composites.

Radial lines are drawn in Fig. 2 to define radial vectors 96 through 101, through which x-rays radiate from the source 24 and impinge upon the sections 70 and 72 of the detectors. X-rays within the radial vectors 96 and 99 do not suffer any attenuation from the ring 66. Thusly, the detectors 74, 75, 82, and 83 will register the strongest signals based upon a positional relationship between the ring 66 and the collar 60 shown in Fig. 2. Conversely, x-rays passing through the radial vectors 97 and 100 and impinging upon detectors 76-78, and 84-86 will show the greatest amount of attenuation. The attenuation will increase from detector 76 through detector 77 and be the greatest in detector 78. Likewise, the attenuation will increase and therefore the received signal will decrease from detector 84 through detector 85 until the weakest signal is received by detector 86. The attenuation increases as the x-rays must pass through succeedingly larger and larger portions of the reference ring 66.

If the relative position of the ring 66 and collar 60 remains the same, the readings from these detectors will likewise remain fairly constant. If, however, the position changes, then the detectors will indicate this change in a measurable manner. For example, if the ring were shifted to the left relatively to the collar 60, the detectors 74 and 75 would show a marked decrease in the received signal as it would undergo attenuation from the ring 66. Likewise, detectors 84, 85, etc. would show a decrease in the amount of attenuation through a corresponding increase in the signal levels produced thereby. Likewise, if the ring 66 were to move relatively downward with respect to the collar 60, both the detectors 76 and 84 would show an increase of output signal corresponding to a decrease in attenuation. Even if the reference ring were to move relative to the collar 60 upwardly and slightly to the right, whereby the detectors 74 through 81 would show no change in their output signals, the detectors 82 and 83 would show a relative decrease in output signals indicating attenuation by the reference ring. Likewise, the other detectors 84 through 89 would also show changes in their respective output signals. It should be noted that this explanation is based upon movement of the reference ring 66. Although this is the movement which is detected by the detectors, the actual relative movement would typically be caused by misalignment of the collar 60 due to its rotation.

The embodiment of Fig. 2 also allows the inclusion of means for enabling a determination of scan quality to be made from images backprojected from the collected scan data. The ring 66 may include a plurality of imaging targets 104. These targets 104 may simply have a known shape, such as circular and/or their x-ray density and the x-ray density of the ring 66 may be known. Whereas prexisting CAT scanners typically blanked images of surrounding structure in the backprojection process, images of the ring 66 and targets 104 are reproduced for the present embodiment to provide, on the actual x-ray, an indication of scan quality. If just the shape of the targets is used, the sharpness of that shape will indicate the accuracy of the scan data collected. In this case it would only be necessary for the targets 104 to have a different though unknown x-ray density than the ring 66. In the event that the x-ray densities of the targets 104 and ring 66 were known, further information would be available with respect to x-ray intensity for evaluating the effectiveness of tissue versus bone imaging.

The ring 66 may also be used to povide a measurement of the angular position between the patient 62 and the collar 60. This may be done by providing some detectable pattern in the x-ray density of the ring 66. The ring may be provided with additional targets 104 or by giving the ring 66 a scallop shape as shown in the section 105 thereof. Such a shape could be detectable by the detector sections 70, 72 to provide a precise angular indication throughout the scanning process.

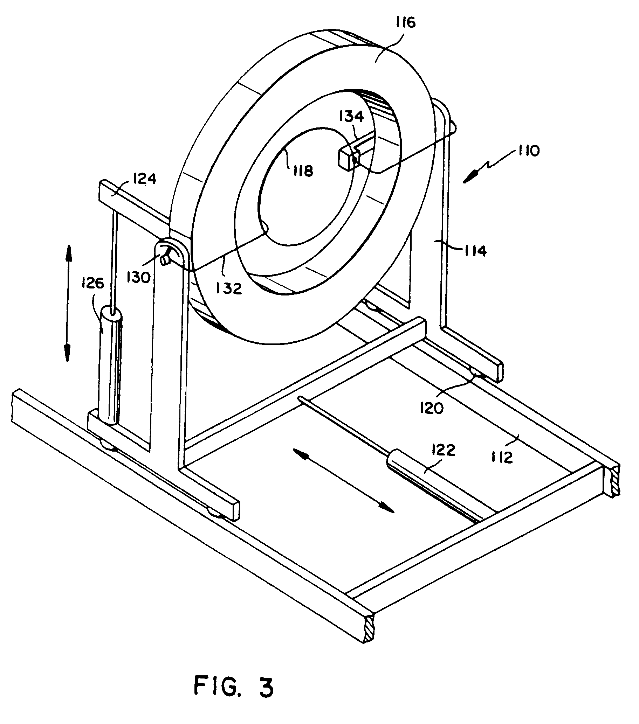

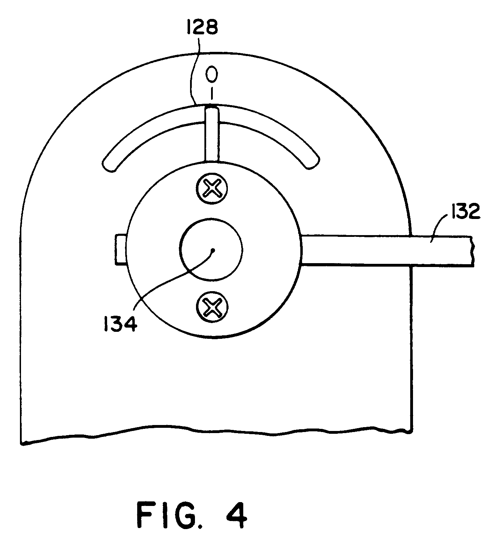

Figs. 3 and 4 show a further refinement of the present invention. An x-ray apparatus 110 is representationally shown having a base 112, a support structure 114, an x-ray component member 116 and a reference ring 118. The support structure 114 is moveably mounted on base 112 by means of rollers 120, and its position along base 112 is controlled by an actuator 122. Actuator 122 may take any suitable form such as hydraulic or electrical.

The x-ray component member 116 may carry an x-ray source and an array of detectors (not shown) along with any other suitable components as previously discussed. The member 116 is rotatably mounted to the support structure 114 to allow angulation of the CAT scan process with respect to a patient, for such purposes as x-raying the spine. Affixed to the member 116 is a manipulator arm 124 which is controlled by an actuator 126 to determine the angular position of the member 116.

The reference ring 118 is likewise rotatably mounted to the support structure 114. Reference ring 118 may be angularly rotated around the same horizontal axis as the component member 116. This enables the ring 118 to be used at any scan angle desired for the member 116. Further, the position of the ring 118 may be electrically sensed and signals therefrom used to control the actuator 126. By this means, the ring 118 may be located by hand, causing the member 116 to follow to the selected position. A sensor may be constructed in any suitable manner. Fig. 4 shows an enlarged view of a potentiometer 128 which may be located at the upper end 130 of support structure 114. A support member 132 for the ring 118 is pivotally mounted at the horizontal axis 134 and is affixed to the sliding contact of a potentiometer 128. By this means, the angular position of the ring 118 as measured by the potentiometer 128 may be used to control the actuator 126 by any suitable control circuitry. Also, the potentiometer 128 may be mounted on the component ring and connected across an electrical bridge network so that an output signal is formed unless the member 116 and ring 118 are in alignment. Such a signal could then be used through suitable control mechanisms to power the actuator 126. In this manner, the ring 118 may be used to detect the relative position of the ring 118 to the member 116 in the dimension substantially perpendicular to the imaginary plane of rotation of the member 116.

Lastly, a plurality of sensors 134 may be mounted on the component member 116 to sense the position of the ring during rotational scanning. These sensors may work both within the plane of rotation of the component member 116 as discussed above and in the dimension substantially perpendicular thereto. They may also provide measurement of the rotational position of the member 116. Further, they may use a combination of different sensing techniques.

The present invention allows a CAT scan apparatus to be constructed which provides accurate scan data without the use of mass and bulk associated with the prior art. With respect to misalignment within the scan plane, deviations may be measured and the results used to improve the accuracy of the collected data. Misalignment of the plane of rotation of the x-ray components may be used to either correct the alignment and/or to disqualify the collected data. Rotational measurement may also be provided in lieu of additional apparatus. These measurements obviate the mass and bulk of the prior art to greatly reduce the cost and mobility of the apparatus.

## Claims

1. A tomography X-ray apparatus having (a) rotatable support means (12) for supporting at least an X-ray source (24) for rotation during a tomography scan of a patient, and (b) X-ray detector means (26) for sensing X-rays during the tomographic scan, said apparatus further comprising:
stationary reference means (42) positioned relative to said rotatable support means so as to remain stationary as said rotatable support means rotates about said axis of rotation during a scan;
position detecting means (40) measuring the positional relationship between the rotatable support means and the stationary reference means; and
generating means for generating data representative of the positional relationship between the rotatable support means and the stationary reference means so that said data can be used when backprojecting a scanned image of the object; characterised in that the stationary reference means is a ring, and in real time during a scan of the patient both the position detecting means measures the positional relationship between the rotatable support means and stationary reference means, and the generating means generates data representative of the said positional relationship.

2. The tomography X-ray apparatus of claim 1. wherein the rotatable support means (12) defines a plane of rotation, and further wherein the positional relationship is determined for the two orthogonal dimensions within the plane of rotation.

3. The tomography X-ray apparatus of claim 1 or claim 2, wherein the rotatable support means is a rotatable collar (12) which defines a first imaginary plane of rotation, and further wherein the ring (42) lies within a second imaginary plane which is either substantially parallel to or coextensive with said first imaginary plane.

4. The tomography X-ray apparatus of claim 3, wherein said position detecting means includes two position detectors (40) for detecting the position of the ring within the second imaginary plane in two orthogonal dimensions.

5. The tomography X-ray apparatus of claim 4, wherein said position detecting means includes a third position detector (128) for detecting the position of the ring in the dimension substantially perpendicular to the second imaginary plane.

6. The tomography X-ray apparatus of claim 5, further comprising means (126) for determining positioning of the collar in the dimension substantially perpendicular to the first plane of rotation in response to the third position detecting means.

7. The tomography X-ray apparatus of claim 4 or claim 5, wherein the position detectors (40) measuring the positional relationship include X-ray detectors (70-72) affixed to the collar and adapted to sense X-rays from the X-ray source to determine the position of the ring.

8. The tomography X-ray apparatus in claim 7, wherein the ring (66) is X-ray translucent and includes means (104) for enabling observation of scan quality from a resulting image.

9. The tomography X-ray apparatus of claim 8, wherein the means for enabling includes target means (106) having a known shape.

10. The tomography X-ray apparatus of claim 8 wherein the means for enabling includes target means (106) having a known X-ray density.

11. The tomography X-ray apparatus of claim 4, wherein the position detector means (40) and said generating means for generating said data includes a pair of mechanical position detectors including means (48) for providing an electrical signal in response to the mechanically detected position of the ring and representative of said data.

12. The tomography X-ray apparatus of claim 11, wherein the mechanical position detectors (60) are affixed to the collar (12) and the ring is affixed to the stationary reference means (16).

13. The tomography X-ray apparatus of claim 4 or claim 5, wherein said position detecting means includes a fourth detector (105) for detecting the angular position of the collar (12) with respect to the ring (42).

14. The tomography X-ray apparatus of claim 1 or claim 2, wherein the stationary reference means includes a patient table (64) means and the ring (42) is affixed to the patient table means.

15. The tomography X-ray apparatus of claim 1 or claim 2, wherein the position detecting means includes at least one source of reflective energy (56) and at least one detector for the reflective energy.

16. The tomography X-ray apparatus of claim 1, wherein the rotatable support means (12) defines a plane of rotation, and wherein said position detecting means (40) and said means for generating said data includes detector means disposed in said plane of rotation for measuring said positional relationship and for generating electrical signal representative of said data.

## Patentansprüche

1. Röntgenstrahlen-Tomographiegerät mit (a) einer rotierbaren Halteeinrichtung (12) zur Halterung zumindest einer Röntgenstrahlungsquelle (24) zur Rotation während einer tomographischen Durchstrahlung eines Patienten, und (b) einer Röntgenstrahlungsdetektoreinrichtung (26) zum Erfassen der Röntgenstrahlung während der tomographischen Durchstrahlung, wobei das Gerät weiterhin umfaßt: eine stationäre Referenzeinrichtung (42), die relativ zur
drehbaren Halteeinrichtung so angeordnet sind, daß sie stationär bleibt, wenn sich die drehbare Halteeinrichtung während der Durchstrahlung um die Rotationsachse dreht;
eine Ortungsdetektoreinrchtung (40) zum Messen der Relativposition zwischen der drehbaren Halteeinrichtung und der stationären Referenzeinrichtung; und
eine Erzeugungseinrichtung zur Erzeugung von Daten über die Relativposition zwischen der drehbaren Halteeinrichtung und der stationären Referenzeinrichtung, so daß die Daten dann verwendet werden können, wenn ein aufgenommenes Bild rückprojiziert wird, **dadurch gekennzeichnet**, daß die stationäre Referenzeinrichtung ein Ring ist, und während der Durchstrahlung eines Patienten sowohl in Echtzeit die Ortungsdetektoreinrichtung die Relativposition zwischen der drehbaren Halteeinrichtung und der stationären Referenzeinrichtung mißt als auch die Erzeugungseinrichtung Daten über die Relativposition erzeugt.

2. Röntgenstrahlen-Tomographiegerät nach Anspruch 1, **dadurch gekennzeichnet**, daß die drehbare Halteeinrichtung (12) eine Rotationsebene definiert, und die Relativposition für die beiden orthogonalen Dimensionen in der Rotationsebene bestimmt werden.

3. Röntgenstrahlen-Tomographiegerät nach Anspruch 1 oder 2, **dadurch gekennzeichnet**, daß die drehbare Halteeinrchtung ein drehbarer Lagerring (12) ist, der eine erste gedachte Rotationsebene definiert und der Ring (42) innerhalb einer zweiten gedachten Ebene liegt, die entweder im wesentlichen parallel zu der ersten Rotationsebene oder in dieser selbst angeordnet ist,

4. Röntgenstrahlen-Tomographiegerät nach Anspruch 3, **dadurch gekennzeichnet**, daß die Ortungsdetektoreinrichtung zwei Ortungsdetektoren (40) zur Erfassung der Position des Rings innerhalb der zweiten gedachten Ebene in zwei orthogonlaen Dimensionen aufweist.

5. Röntgenstrahlen-Tomographiegerät nach Anspruch 4, **dadurch gekennzeichnet**, daß die Ortungsdetektoreinrichtung einen dritten Ortungsdetektor (128) zur Erfassung der Position des Rings in der im wesentlichen zur zweiten gedachten Ebene senkrechten Dimension aufweist.

6. Röntgenstrahlen-Tomographiegerät nach Anspruch 5, **gekennzeichnet durch** eine Einrichtung (126) zur Positlonierung des Lagerrings in der im wesentlichen zur ersten Rotationsebene senkrechten Dimension in Abhängigkeit vom dritten Ortungsdetektor.

7. Röntgenstrahlen-Tomographiegerät nach Anspruch 4 oder 5, **dadurch gekennzeichnet**, daß die die Relativposition messenden Ortungsdetektoren (40) Röntgenstrahlungsdetektoren (70-72) aufweisen, die am Lagerring angebracht und dazu ausgelegt sind, die von der Röntgenstrahlungsquelle ausgesandten Röntgenstrahlen zur Ortung des Rings zu erfassen.

8. Röngenstrahlen-Tomographiegerät nach Anspruch 7, **dadurch gekennzeichnet**, daß der Ring (66) für Röntgenstrahlen durchlässig ist und eine Einrichtung (104) zur Überwachung der Abbildungsqualität von einem resultierenden Bild aufweist.

9. Röntgenstrahlen-Tomographiegerät nach Anspruch 8, **dadurch gekennzeichnet**, daß die Einrichtung zur Überwachung der Abbildungsqualität Abbildungsprobenmittel (104) mit bekannter Form aufweist.

10. Röntgenstrahlen-Tomographiegerät nach Anspruch 8, **dadurch gekennzeichnet**, daß die Einrichtung zur Überwachung der Abbildungsqualität Abbildungsprobenmittel (105) aufweist, deren Röntgenstrahlungsdurchlässigkeit bekannt ist.

11. Röntgenstrahlen-Tomographiegerät nach Anspruch 4, **dadurch gekennzeichnet**, daß die Ortungsdetektoreinrichtung (40) und die Erzeugungseinrichtung zur Erzeugung der Daten ein Paar mechanische Ortungsdetektoren aufweisen, die eine Einrichtung (48) zur Bereitstellung eines elektrischen Signals in Abhängigkeit der mechanisch erfaßten Position des Rings aufweisen, wobei das Signal die Daten darstellt.

12. Röntgenstrahlen-Tomographiegerät nach Anspruch 11, **dadurch gekennzeichnet**, daß die mechanischen Ortungsdetektoren (60) am Lagerring (12) angebracht sind und der Ring an der stationären Referenzeinrichtung (16) befestigt ist.

13. Röntgenstrahlen-Tomographiegerät nach Anspruch 4 oder 5, **dadurch gekennzeichnet,** daß die Ortungsdetektoreinrichtung einen vierten Detektor (105) zur Erfassung der relativen Winkelposition des Lagerrings (12) zu dem Ring (42) aufweist.

14. Röntgenstrahlen-Tomographiegerät nach Anspruch 1 oder 2, **dadurch gekennzeichnet**, daß die stationäre Referenzeinrichtung einen Patiententisch (64) aufweist, an dem der Ring (42) angebracht ist.

15. Röntgenstrahlen-Tomographiegerät nach Anspruch 1 oder 2, **dadurch gekennzeichnet**, daß die Ortungsdetektoreinrichtung wenigstens eine Quelle reflektierbarer Energie (56) und wenigstens einen Detektor für die reflektierbare Energie aufweist.

16. Röntgenstrahlen-Tomographiegerät nach Anspruch 1, **dadurch gekennzeichnet**, daß die drehbare Halteeinrichtung (12) eine Rotationsebene definiert, und die Ortungsdetektoreinrichtung (40) und die Einrichtung zur Erzeugung der Daten Detektoren aufweisen, die in der Rotationsebene zur Messung der Relativposition und zur Erzeugung eines die Daten darstellenden elektrischen Signals angeordnet sind.

## Revendications

1. Appareil de tomographie par rayons X comprenant (a) un support tournant (12) pour supporter au moins une source de rayons X (24) en rotation pendant un balayage tomographique d'un patient, et (b) des moyens de détection de rayons X (26) pour détecter les rayons X pendant le balayage tomographique, ledit appareil comprenant en outre :
des moyens de référence stationnaires (42) positionnés par rapport audit support tournant de façon à rester fixes lorsque ledit support tournant tourne autour dudit axe de rotation pendant un balayage :
des moyens de détection de position (40) mesurant la relation de position entre le support tournant et les moyens de référence stationnaires ; et
des moyens de génération pour engendrer des données représentatives de la relation de position entre le support tournant et les moyens de référence stationnaires de sorte qu'on peut utiliser lesdites données lors de la rétroprojection d'une image de balayage de l'objet ;
caractérisé en ce que les moyens de référence stationnaires sont un anneau et, en temps réel pendant un balayage du patient, à la fois les moyens de détection de position mesurent la relation de position entre le support tournant et les moyens de référence stationnaires, et les moyens de génération engendrent des données représentatives de ladite relation de position.

2. Appareil de tomographie par rayons X selon la revendication 1, dans lequel le support tournant (12) définit un plan de rotation, et en outre dans lequel la relation de position est déterminée pour les deux dimensions orthogonales dans le plan de rotation.

3. Appareil de tomographie par rayons X selon la revendication 1 ou la revendication 2, dans lequel le support tournant est un collier tournant (12) qui définit un premier plan imaginaire de rotation, et en outre dans lequel l'anneau (42) est dans un deuxième plan imaginaire qui est sensiblement parallèle audit premier plan imaginaire ou coextensif avec celui-ci.

4. Appareil de tomographie par rayons X selon la revendication 3, dans lequel lesdits moyens de détection de position comprennent deux détecteurs de position (40) pour détecter la position de l'anneau dans le deuxième plan imaginaire, dans deux dimensions orthogonales.

5. Appareil de tomographie par rayonz X selon la revendication 4, dans lequel lesdits moyens de détection de position comprennent un troisième détecteur de position (128) pour détecter la position de l'anneau dans la dimension sensiblement perpendiculaire au deuxième plan imaginaire.

6. Appareil de tomographie par rayons X selon la revendication 5, comprenant en outre des moyens (126) pour déterminer le positionnement du collier dans la dimension sensiblement perpendiculaire au premier plan de rotation un réponse au troisième détecteur de position.

7. Appareil de tomographie par rayons X selon la revendication 4 ou la revendication 5, dans lequel les détecteurs de position (40) mesurant la relation de position comprennent des détecteurs de rayons X (70-72) fixés au collier et pouvant détecter les rayons X émis par la source de rayons X pour déterminer la position de l'anneau.

8. Appareil de tomographie par rayons X selon la revendication 7, dans lequel l'anneau (66) est translucide aux rayons X et comprend des moyens (104) pour permettre l'observation de la qualité de d'analyse d'une image résultante.

9. Appareil de tomographie par rayons X selon la revendication 8, dans lequel les moyens permettant l'observation comprennent des cibles (106) de forme connue.

10. Appareil de tomographie par rayons X selon la revendication 8, dans lequel les moyens permettant l'observation comprennent des cibles (106) ayant une densité connue aux rayons X.

11. Appareil de tomographie par rayons X selon la revendication 4, dans lequel les moyens de détection de position (40) et lesdits moyens de génération pour engendrer lesdites données comprennent deux détecteurs de position mécaniques comportant des moyens (48) de fourniture d'un signal électrique en réponse à la position mécaniquement détectée de l'anneau et représentatif desdites données.

12. Appareil de tomographie par rayons X selon la revendication 11, dans lequel les détecteurs de position mécaniques (60) sont fixés au collier (12) et l'anneau est fixé aux moyens de référence stationnaires (16).

13. Appareil de tomographie par rayons X selon la revendication 4 ou la revendication 5, dans lequel lesdits moyens de détection de position comprennent un quatrième détecteur (105) pour détecter la position angulaire du collier (12) par rapport à l'anneau (42).

14. Appareil de tomographie par rayons X selon la revendication 1 ou la revendication 2, dans lequel les moyens de référence stationnaires comprennent une table de patient (64) et l'anneau (42) est fixé a la table de patient.

15. Appareil de tomographie par rayons X selon la revendication 1 ou la revendication 2, dans lequel les moyens de détection de position comprennent au moins une source d'énergie réflective (56) et au moins un détecteur de l'énergie réfléchie.

16. Appareil de tomographie par rayons X selon la revendication 1, dans lequel le support tournant (12) définit un plan de rotation, et dans lequel les dits moyens de détection de position (40) et les dits moyens de génération de données comprennent des déflecteurs disposés dans ledit plan de rotation pour mesurer la dite relation de position et engendrer un signal électrique représentatif desdites données.
